# EUROPEAN PATENT APPLICATION

(11) **EP 2 371 315 A1**
(43) Date of publication of application: **05.10.2011**
(21) Application number: 11152239.7
(22) Date of filing: 26.01.2011
(51) Int. Cl.: A61B 18/14

(54) **Endoscopic high-frequency treatment instrument**

(30) Priority: 31.03.2010 JP 2010084416
(71) Applicant: Fujifilm Corporation, Minato-ku Tokyo (JP)
(72) Inventor: Akahane, Hidefumi, Kanagawa (JP)
(74) Representative: Klunker . Schmitt-Nilson . Hirsch

(57) **Abstract**

An endoscopic high-frequency treatment instrument includes a sheath, an operation wire, a first gripping part, a second gripping part and a first electrode. The sheath includes a front end, a rear end, and a longitudinal shaft. The operation wire is provided in the sheath so as to advance and retreat. The first gripping part is fixed to extend from the front end of the sheath along the longitudinal shaft. The second gripping part is connected to the operation wire and is moved about a base end thereof as a rotation center so as to approach and be separated from the first gripping part as the operation wire advances and retreats with respect to the sheath. The first electrode is provided on a surface of the first gripping part which faces the second gripping part.

## Description

### BACKGROUND OF THE INVENTION

### 1. Technical Field

The present invention relates to an endoscopic high-frequency treatment instrument.

### 2. Description of Related Art

In medical diagnosis and treatment using an endoscope, the treatment instrument is inserted into the forceps hole of the endoscope and can make incisions in, or coagulate, the body tissue of a desired portion in a body cavity, as well as performing various treatments such as harvesting body tissue, extracting an affected area, and the like. As high-frequency treatment instruments inserted into the forceps hole of an endoscope to perform treatments such as incising or coagulating tissue, such as that disclosed in JP-A-2007-312889, many such instruments have been proposed.

However, great skill is required in controlling the direction of incision, as a high-frequency treatment instrument is able to incise an affected area instantly and easily. Care must therefore be taken to avoid inadvertently incising the deep layer (muscle layer and the like) of tissue other than the affected area.

Further, when using an endoscope to treat an affected area, different kinds of treatment instruments are used depending on the type of treatment. Such treatment instruments are sequentially inserted into the forceps hole at the required time, and the respective treatments are performed individually. However, when the above-mentioned treatments are actually performed using an endoscope, there can often be a multiplicity of treatment procedures. For this reason, the time, which is taken to insert the treatment instrument into the forceps hole and to take the treatment instrument from the forceps hole, is too much other than the time taken for the treatments themselves. Accordingly, the time, which is taken to insert the treatment instrument into the forceps hole and to take the treatment instrument out from the forceps hole, cannot be ignored. For example, if bleeding occurs from the affected area after a high-frequency knife is inserted into the forceps hole to make an incision, the high-frequency knife must be extracted from the forceps hole of the endoscope and replaced by newly inserting a high-frequency coagulation instrument to perform hemostasis at the bleeding portion. As a result, the handling of treatment instruments is problematic.

### SUMMARY

An object of the invention is to provide an endoscopic high-frequency treatment instrument, which can easily control a treatment for incising body tissue and also perform other treatments using a high-frequency electrode, as a high-frequency treatment instrument that uses a gripping part as a high-frequency electrode, so as to more rapidly simplify a treatment.

An endoscopic high-frequency treatment instrument includes a sheath, an operation wire, a first gripping part, a second gripping part and a first electrode. The sheath includes a front end, a rear end, and a longitudinal shaft. The operation wire is provided in the sheath so as to advance and retreat. The first gripping part is fixed to extend from the front end of the sheath along the longitudinal shaft. The second gripping part is connected to the operation wire and is moved about a base end thereof as a rotation center so as to approach and be separated from the first gripping part as the operation wire advances and retreats with respect to the sheath. The first electrode is provided on a surface of the first gripping part which faces the second gripping part.

According to the endoscopic high-frequency treatment instrument of the invention, when the electrode of the stationary gripping part acts selectively only on the surface of the body tissue that exists in the moving direction during the incision and the direction of incision of the body tissue nears the deep layer (muscle layer) of tissue, the movable gripping part receives a reaction force from the body tissue and is stopped. Accordingly, the protruding body tissue is incised in a direction parallel to the peripheral body tissue and the incision is prevented from extending toward the deep layer (muscle layer) of tissue. Further, hemostasis is performed by holding the body tissue by the gripping parts, that is, it may be possible to continuously perform a treatment without inserting the treatment instrument into the forceps hole of an endoscope and taking the treatment instrument out from the forceps hole of an endoscope.

Furthermore, according to the endoscopic high-frequency treatment instrument of the invention, it may be possible to increase a range where the incision can be performed and to increase the range where the electrodes are provided by the cooperation of the stationary electrode of the stationary gripping part and the movable electrode of the movable gripping part.

### BRIEF DESCRIPTION OF THE DRAWINGS

Fig. 1 is a view showing the entire configuration of an endoscopic high-frequency treatment instrument that is used to illustrate an embodiment of the invention.
Fig. 2 is a cross-sectional view showing an opening/closing mechanism of the gripper and the connection portion between the insertion portion and the operating unit.
Fig. 3 is an enlarged view of the gripper in a state where a movable gripping part is opened at an angle of 90° to the stationary gripping part.
Fig. 4 is a schematic cross-sectional view taken along a line A-A of Fig. 2.
Fig. 5 is a side view showing a state where the stationary gripping part is guided by the movable gripping part coming into contact with the surface of body tissue.
Fig. 6 is a plan view showing a state where a mucosal lesion region is marked.
Fig. 7 is a cross-sectional view of tissue when local injection is administered to a mucosal lesion region.
Fig. 8 is a perspective view showing a state where a mucosal lesion region is incised by the stationary gripping part while being guided by the movable gripping part.
Fig. 9A is a cross-sectional view of a gripper of a first modification and Fig. 9B is a cross-sectional view of a gripper of a second modification.
Fig. 10A is a side view of a gripper of a third modification and Fig. 10B is a bottom view.
Fig. 11 is an enlarged view of a gripper of an endoscopic high-frequency treatment instrument as another example.
Fig. 12 is a side view of a gripper of a fourth modification.
Fig. 13A is an enlarged view of the tip portion of a stationary gripping part of a fifth modification and Fig. 13B is an enlarged view of the tip portion of a stationary gripping part of a sixth modification.

### DETAILED DESCRIPTION OF EMBODIMENTS OF THE INVENTION

The embodiments of the invention will be described in detail below with reference to the drawings.

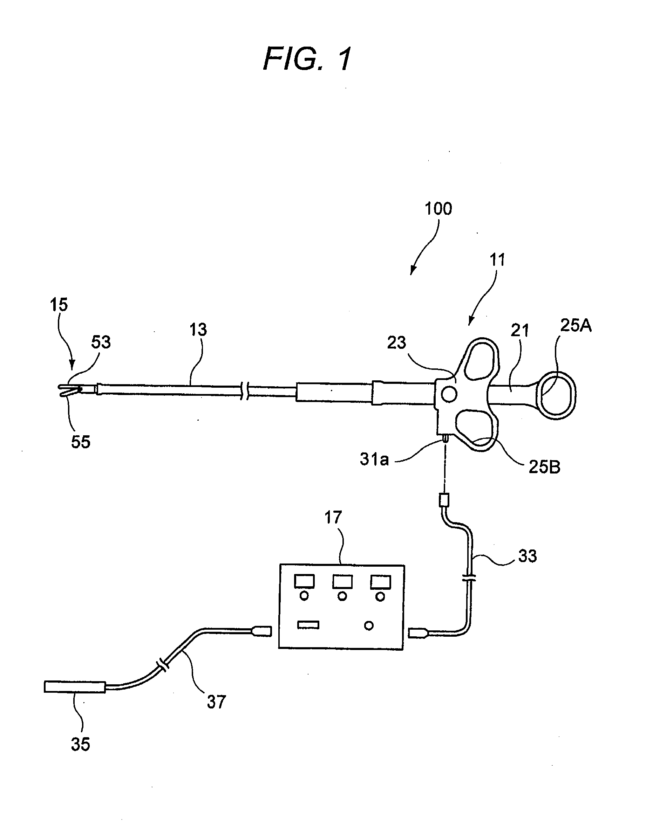

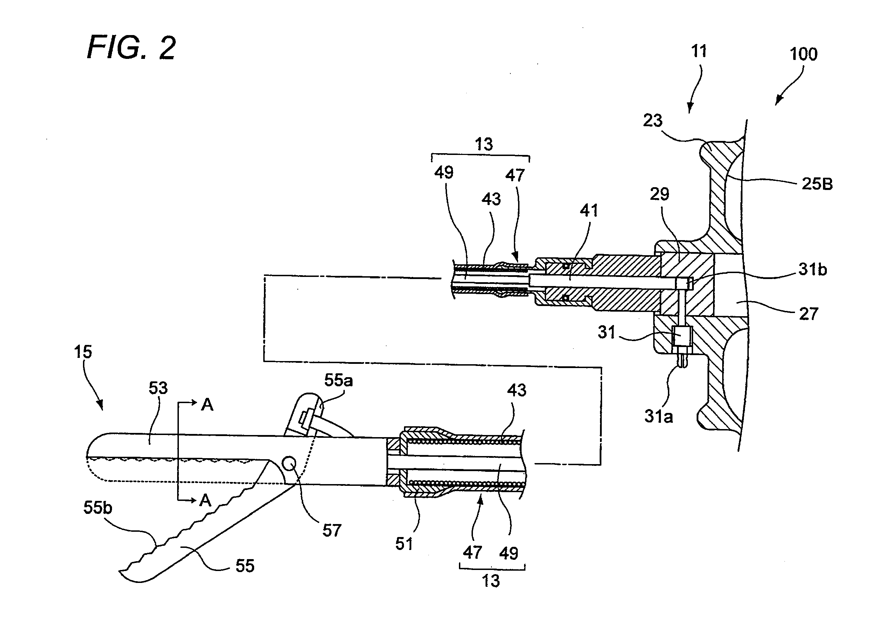

Fig. 1 is a view showing the entire configuration of the endoscopic high-frequency treatment instrument used to illustrate an embodiment of the invention, and Fig. 2 is a cross-sectional view showing an opening/closing mechanism of the gripper and the connection portion between the insertion portion and the operating unit.

As shown in Fig. 1, the endoscopic high-frequency treatment instrument 100 is a high-frequency treatment instrument that includes the operating unit 11, the insertion portion 13 extending from the operating unit 11, one gripper 15 provided at the tip of the insertion portion 13, and the high-frequency power source 17. The endoscopic high-frequency treatment instrument 100 is formed of a monopolar high-frequency treatment instrument that makes high-frequency current flow while the gripper 15 is inserted into the body cavity and a counter electrode 35 to be described below comes into contact with the epidermis of a patient.

The operating unit 11 includes a shaft portion 21 where a finger hooking portion 25A is provided and a slider 23 where a finger hooking portion 25B is provided. The slider 23 is fitted to the shaft portion 21. As shown in Fig. 2, the slide piece 29 connected to the slider 23 is installed in a slit 27 formed by the shaft portion 21. The terminal portion 31, where the external terminal 31a is attached to face the outside of the slider 23, is provided at the slide piece 29. As shown in Fig. 1, the external terminal 31a of the terminal portion 31 is connected to the high-frequency power source 17 by the cable 33. Further, the cord 37 of the counter electrode 35, which comes into contact with the epidermis of a patient, is connected to the high-frequency power source 17.

The terminal portion 31 is connected to the slide member 41 as shown in Fig. 2, and the slide member 41 is connected to an operation wire 49 of the insertion portion 13. The insertion portion 13 includes a flexible sheath 47 and the operation wire 49. The flexible sheath 47 is formed by coating the coil sleeve 43, which is formed of a closely wound coil, with an insulating tube (not shown). The operation wire 49 is slidably inserted into the flexible sheath 47 in the axial direction. Accordingly, the insertion portion 13 has softness and flexibility as a whole. The flexible sheath 47 includes a front end, a rear end, and a longitudinal shaft. The operation wire 49 is formed of a conductive wire, and the surface thereof is covered with an insulating coating if necessary.

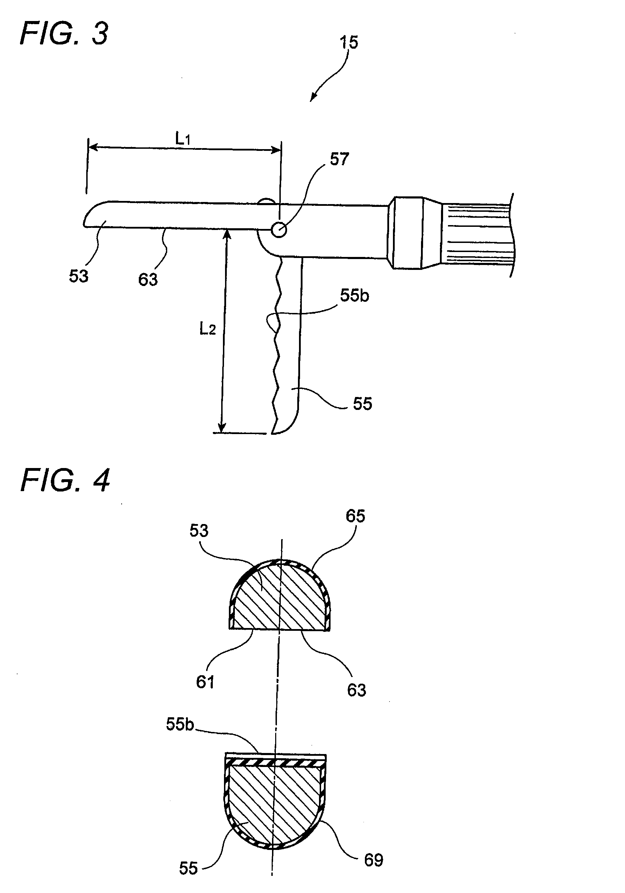

Fig. 3 is an enlarged view of the gripper, and shows a state where the movable gripping part (second gripping part) 55 is opened at an angle of 90° to the stationary gripping part (first gripping part) 53. The gripper 15 is placed at the tip of the insertion portion 13. The stationary gripping part 53, which extends in the axial direction of the insertion portion 13, is fixed to the connection member 51 fixed to the tip of the flexible sheath 47. The movable gripping part 55 is rotatably fitted to the support shaft 57 that is placed at a position retreating toward the base end from the tip of the stationary gripping part 53. The operation wire 49 is connected to one end 55a of the movable gripping part 55, which is close to the operating unit 11, through an operation member 59, so that an opening/closing mechanism is formed.

A serrated concavo-convex portion 55b where convex portions having acuate tips and concave portions are alternately provide is formed on the inner surface of the movable gripping part 55, that is, the surface of the movable gripping part 55 facing the stationary gripping part 53. The serrated concavo-convex portion 55b is formed over the entire length of the movable gripping part 55 in the width direction thereof so as to be capable of reliably gripping the body tissue.

Accordingly, when the slider 23 is moved in the axial direction of the shaft portion 21, the slide piece 29 is also moved in the axial direction at the same time. This causes the operation wire 49 connected to the slide piece 29 to be pushed and pulled inside the flexible sheath 47. As a result, the operation member 59 is moved in the axial direction and the movable gripping part 55 is opened and closed on one side in relation to the stationary gripping part 53.

The opening angle of the movable gripping part 55 about the support shaft 57 is up to a right angle, or up to a range in which the angle is an obtuse angle. The distance L1 between the tip of the stationary gripping part 53 and the rotation center (support shaft 57) is set to be equal to, or slightly larger than, the distance L2 between the tip of the movable gripping part 55 and the rotation center (support shaft 57). When the movable gripping part 55 is closed, the inner surfaces of the movable gripping part 55 and the stationary gripping part 53, that is, the surfaces facing each other, come into contact with each other and grip the body tissue so that the body tissue is interposed between both the gripping parts 53 and 55.

The terminal portion 31 is electrically connected to the stationary gripping part 53 through the connecting portion 31b, the slide member 41, the operation wire 49, and the operating member 59. Each of these members is formed of a conductive member, and these members are covered with an insulating coating except for contact portions between the members. Accordingly, a power supply path, which reaches the gripping part 53 from the terminal portion 31 formed at the slider 23, is formed through these parts.

Fig. 4 is a schematic cross-sectional view taken along a line A-A of Fig. 2. The stationary gripping part 53 is formed of a metal base such as stainless steel, and is formed to have a substantially semicircular cross-section so that the surface of the stationary gripping part 53, which faces the movable gripping part 55 when the gripper 15 is closed, is formed of a flat surface 61. The flat surface 61 is a conductive surface, that is, a blade surface on which the electrode (first electrode) 63 is formed. The surface of the stationary gripping part 53 except for the flat surface 61 is formed of a non-conductive surface on which the insulating coating 65 made of ceramics, resin, or the like is applied.

Furthermore, the movable gripping part 55 is likewise made of metal such as stainless steel and formed to have a substantially semicircular cross-section. The serrated concavo-convex portion 55b where convex portions and concave portions are alternately formed side by side in the longitudinal direction is formed on the surface of the movable gripping part 55 facing the stationary gripping part 53. The entire surface of the movable gripping part 55 is formed of a non-conductive surface on which the insulating coating 69 is applied. Meanwhile, the movable gripping part 55 may be formed of insulating ceramics such as alumina or zirconia, or may be formed of resins such as polytetrafluoroethylene (PTFE). In addition, the surfaces of the stationary gripping part 53 and the movable gripping part 55 facing each other are flat, but the surface of the stationary gripping part 53 may be formed of a serrated concavo-convex portion that engages with the serrated concavo-convex portion 55b of the movable gripping part 55.

Next, the operation of the endoscopic high-frequency treatment instrument 100 having this structure will be described.

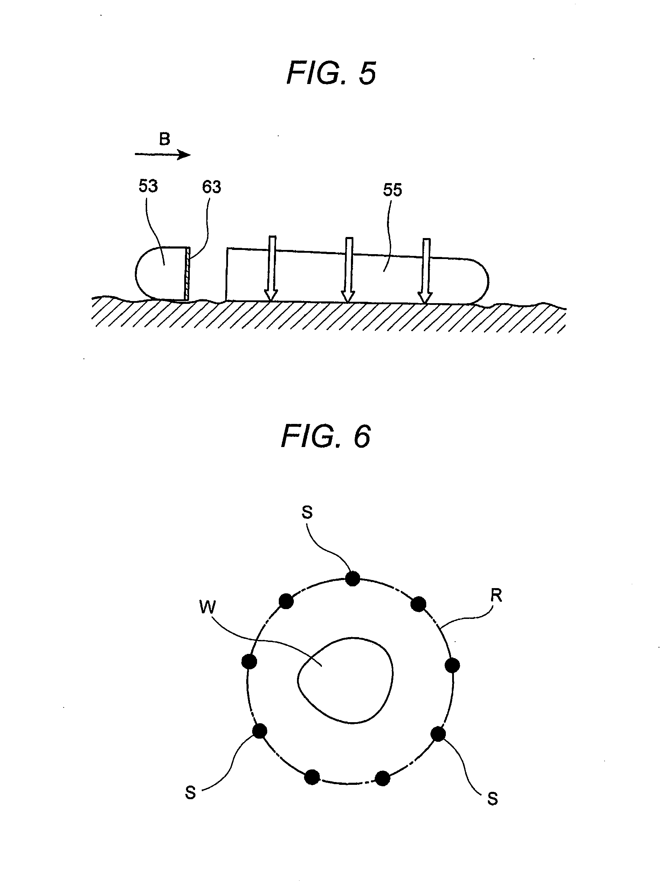

Fig. 5 is a side view of the stationary gripping part being guided by the movable gripping part coming into contact with the surface of body tissue. When high-frequency current flows through the electrode 63 of the stationary gripping part 53 from the high-frequency power source 17 and the body tissue 71 is incised while the movable gripping part 55 is opened on one side at an angle of about 90°, the side surfaces of the stationary gripping part 53 and the movable gripping part 55 are pressed against the surface of the body tissue 71. Accordingly, when the stationary gripping part 53 is moved in the direction of an arrow B, the stationary gripping part 53 is guided in the moving direction thereof by the movable gripping part 55. That is, when the incision direction of the stationary gripping part 53 is directed toward the deep layer (muscle layer) of tissue, the movable gripping part 55 receives a reaction force from the body tissue 71 and is stopped. Accordingly, it may be possible to perform a treatment simply and smoothly without the risk of damaging or perforating the muscle layer.

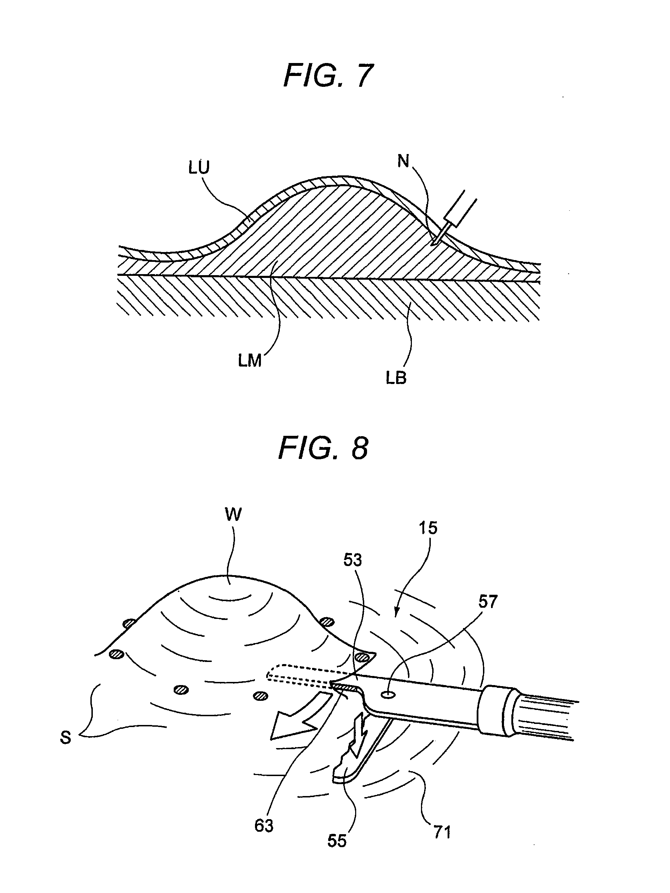

Shown here is an example of ESD (Endoscopic Submucosal Dissection), one of the treatments for excising mucosal lesion from an inner wall of a body cavity, such as the esophagus, the stomach, the duodenum, or the large intestine, by using the endoscopic high-frequency treatment instrument 100 of the above-mentioned structure. The ESD will be described with reference to Figs. 6 to 8. Fig. 6 is a plan view showing a state where a mucosal lesion region is marked. Fig. 7 is a cross-sectional view of tissue when local injection is administered to a mucosal lesion region. Fig. 8 is a perspective view showing a state where a mucosal lesion region is incised by the stationary gripping part while being guided by the movable gripping part.

The ESD is performed when the existence of a lesion portion is confirmed in a mucosa after the inside of the body cavity is examined using an endoscope. Initially, a portion of the mucosa to be excised is marked, and a local injection is administered to swell the portion of mucosal lesion. Subsequently, the mucosa is incised along the marked region by using the endoscopic high-frequency treatment instrument 100, the fiber forming submucosa is cut off, and the mucosa is separated from the muscle layer.

Specifically, the endoscopic high-frequency treatment instrument 100 is inserted into the body cavity through a forceps hole of an endoscope (not shown), and a mucosal lesion region R is set and marked on the mucosa where a lesion portion W to be excised exists so as to surround the lesion portion W as shown in Fig. 6. Marking is performed by setting ablation spots S with the high-frequency treatment instrument at required positions around the mucosal lesion region R. Moreover, marking may be performed in any manner as long as the region of the mucosa to be excised can be recognized using an endoscope, and may be performed using other treatment instruments.

Saline solution, sodium hyaluronate, or the like is then locally injected into the mucosal lesion region R as shown in Fig. 7. For this purpose, the high-frequency treatment instrument for marking is withdrawn from the forceps hole, and a local injection means that includes the injection needle N provided at the tip of a flexible tube is inserted into the forceps hole instead. Here, the submucosa LM exists between the muscle layer LB and the mucosal layer LU, and the injection needle N is inserted into the submucosa LM through the mucosal layer LU and injects saline solution to the submucosa LM. Accordingly, the submucosa LM is swelled and raised up.

Further, after the submucosa LM is sufficiently swelled, the local injection means is taken out from the forceps hole and the endoscopic high-frequency treatment instrument 100 is inserted. Furthermore, the electrode 63 of the endoscopic high-frequency treatment instrument 100 is pressed as shown in Fig. 8, and high-frequency current flows in the electrode 63 from the high-frequency power source 17. Accordingly, the outer peripheral portion of the mucosal lesion region R is incised. As a result, the mucosal layer LU is incised on the outer periphery of the mucosal lesion region R, and the submucosa LM is left exposed.

Next, the stationary gripping part 53 protruding from the insertion portion 13 of the endoscopic high-frequency treatment instrument 100 enters the exposed portion of the submucosa LM, which is formed by the incision, and is moved horizontally or swung, so that the submucosa LM is cut off by the electrode 63.

In this case, the electrode 63 (the stationary gripping part 53) is moved horizontally or swung while the side surface of the movable gripping part 55 opened at an angle of about 90° comes into contact with the surface of the body tissue 71. Accordingly, when the incision direction of the stationary gripping part 53 (electrode 63) is directed toward the deep layer (muscle layer LB) of tissue, the movable gripping part 55 receives a reaction force from the body tissue 71 and is stopped.

The electrode 63 therefore selectively acts only on the surface of the body tissue that exists in the moving direction of the stationary gripping part 53, so that it may be possible to incise the protruding body tissue (mucosal lesion region R) in a direction parallel to the region of other body tissue 71. As a result, the area of incision is prevented from extending toward the deep layer (muscle layer LB) of tissue, so that the muscle layer LB is not damaged.

Moreover, when bleeding occurs due to incision or the like, it is possible to perform hemostasis by holding the body tissue 71 by the stationary gripping part 53 and the movable gripping part 55, applying high-frequency current to the electrode 63, and by ablation followed by coagulation. Further, treatments such as ablation of the body tissue 71 are possible. As described above, the endoscopic high-frequency treatment instrument 100 having this structure combines the functions of a high-frequency knife with a grip function. Accordingly, it is possible to continuously and efficiently perform treatment without inserting other treatment instruments such those for hemostasis into the forceps hole again after taking the endoscopic high-frequency treatment instrument 100 out from the forceps hole of an endoscope. Therefore, it is possible to speedily detach a mucosa.

It is preferable that the opening angle of the movable gripping part 55 with respect to the stationary gripping part 53 be set to roughly 90° when the function to prevent the incision direction of the stationary gripping part 53 from being directed toward the deep layer of the tissue is emphasized. It is preferable that the opening angle of the movable gripping part 55 with respect to the stationary gripping part 53 be set to an obtuse angle, particularly, almost to 180°, when the rapid incision function as a high-frequency knife is emphasized. The opening angle of the movable gripping part 55 may be appropriately set to an arbitrary angle according to the portion where the endoscopic high-frequency treatment instrument 100 is applied (the use of the endoscopic high-frequency treatment instrument 100).

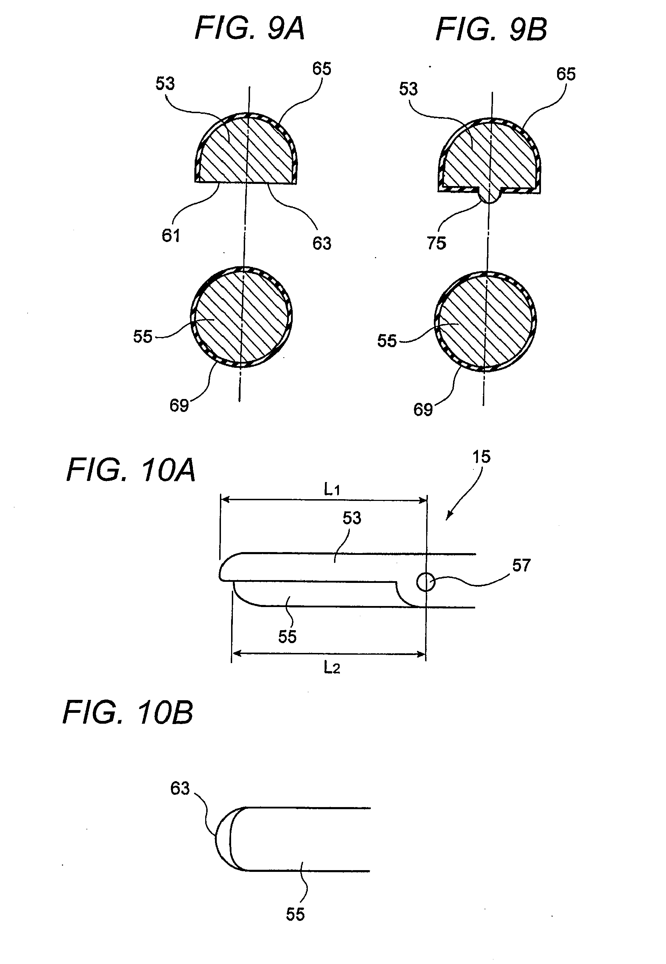

Fig. 9A is a cross-sectional view of a gripper of the first modification, and the movable gripping part 55 is formed to have a circular cross-section. Accordingly, when the movable gripping part 55 is moved along body tissue 71, the possibility that the body tissue 71 is damaged by the movable gripping part 55 is reduced. Fig. 9B is a cross-sectional view of a gripper of the second modification. The movable gripping part 55 has a circular cross-section and an electrode of a stationary gripping part 53 is formed of the protruding electrode portion 75 that protrudes in a tapered shape toward the movable gripping part 55. Since the protruding electrode portion 75 protrudes from the surface of the stationary gripping part 53, the protruding electrode portion 75 increases grip pressure when cooperating with the movable gripping part 55 so as to hold the body tissue 71. Accordingly, the body tissue 71 is reliably gripped and the ablation or incision using high-frequency waves is accurately performed.

Figs. 10A and 10B show a gripper in the third modification; Fig. 10A is a side view, and Fig. 10B is a bottom view. As shown in Figs. 10A and 10B, as for the gripper 15 of the third modification, the distance L1 between the tip of the stationary gripping part 53 and the support shaft 57 (rotation center) is set to be larger than the distance L2 between the tip of the movable gripping part 55 and the support shaft 57 (rotation center). Accordingly, when the gripper 15 is closed, the electrode 63 of the stationary gripping part 53 is exposed to the outside by the difference between the distances L1 and L2.

According to the endoscopic high-frequency treatment instrument, when the gripper 15 is closed, the endoscopic high-frequency treatment instrument can be used as a marking treatment instrument, which sets ablation spots S around the above-mentioned mucosal lesion region R using the electrode 63 exposed to the tip of the stationary gripping part 53.

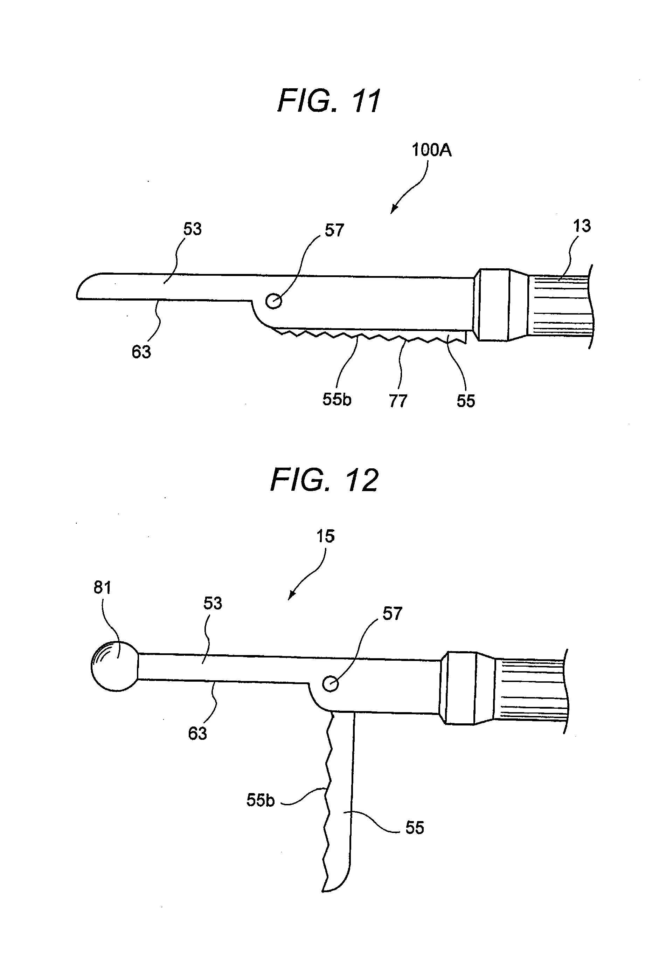

Fig. 11 is an enlarged view of a gripper of an endoscopic high-frequency treatment instrument as another example. The endoscopic high-frequency treatment instrument 100A is different from the above-mentioned endoscopic high-frequency treatment instrument 100 in that the movable electrode (second electrode) 77 is formed on the surface of the movable gripping part 55 facing a stationary gripping part 53 as shown in Fig. 11, in addition to an electrode 63 formed at the stationary gripping part 53. The movable electrode 77 is connected to the high-frequency power source 17, so that high-frequency current can be applied to the movable gripping part 55. The surface of the movable gripping part 55, except for the movable electrode 77, is formed of an insulated, non-conductive surface.

When the movable gripping part 55 is opened by an angle close to 180°, the endoscopic high-frequency treatment instrument 100A can make the stationary electrode 63 of the stationary gripping part 53 and the movable electrode 77 of the movable gripping part 55 cooperate to act and the range where the electrodes 63 and 75 are provided is increased. Accordingly, it is possible to further increase the range in which the incision can be performed. It is therefore possible to perform incisions rapidly and efficiently.

Fig. 12 is a side view of a gripper in the fourth modification. As shown in Fig. 12, the gripper 15 in the fourth modification includes the spherical portion 81, which is made of an insulating material or a metal of which the surface is insulated, at the tip of the stationary gripping part 53. According to the gripper 15, the tip of the stationary gripping part 53 is formed in a spherical shape. Accordingly, any unintended damage of the tissue caused by the tip portion can be prevented. Meanwhile, the spherical portion 81 may be a conductive body, in which case the spherical portion 81 can be used as a knife to make incisions.

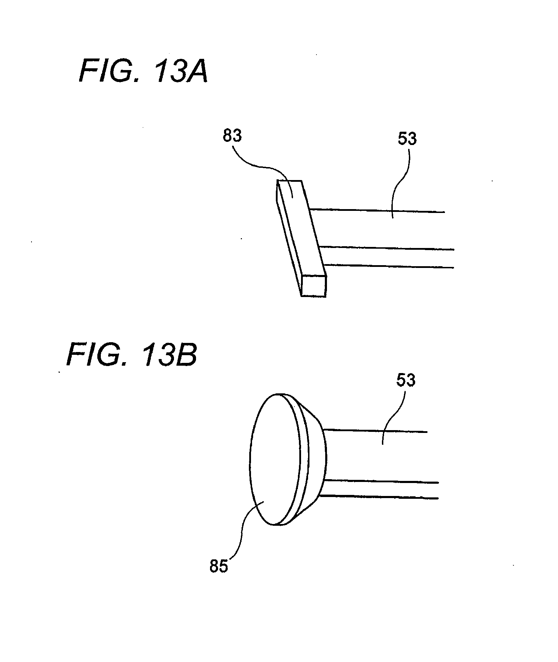

Fig. 13A is an enlarged view of the tip portion of a stationary gripping part in the fifth modification and Fig. 13B is an enlarged view of the tip portion of a stationary gripping part in the sixth modification. As shown in Figs. 13A and 13B, the electrode 83 in the shape of a rectangular parallelepiped hook is formed at the tip of the stationary gripping part 53 in the fifth modification, and a substantially disk-shaped disk electrode 85 is formed at the tip of the stationary gripping part 53 in the sixth modification. These electrodes, which are formed at the tip of the stationary gripping part 53, may have various shapes according to their use, and can be used as a knife to suit various types of treatment.

As described above, the invention is not limited to the above-mentioned embodiment. Further, modifications and applications, which are to be performed by those skilled in the art on the basis of the description of the specification and known techniques, are also anticipated by the invention and are included in the range to be protected.

As described above, the following is disclosed in this specification.
(1) An endoscopic high-frequency treatment instrument includes a sheath, an operation wire, a first gripping part, a second gripping part and a first electrode. The sheath includes a front end, a rear end, and a longitudinal shaft. The operation wire is provided in the sheath so as to advance and retreat. The first gripping part is fixed to extend from the front end of the sheath along the longitudinal shaft. The second gripping part is connected to the operation wire and is moved about a base end thereof as a rotation center so as to approach and be separated from the first gripping part as the operation wire advances and retreats with respect to the sheath. The first electrode is provided on a surface of the first gripping part which faces the second gripping part.

According to the endoscopic high-frequency treatment instrument, the second gripping part is opened on one side with respect to the first gripping part. Accordingly, when the first gripping part and the opened second gripping part are pressed against the body tissue, the first electrode of the first gripping part does not come into direct contact with the surface of the body tissue and the high-frequency current is selectively generated only on the surface of the body tissue that exists in the moving direction. That is, the second gripping part is always disposed toward the incision direction. Accordingly, when the direction where the body tissue is incised is directed toward the deep layer (muscle layer) of tissue during the incision, the second gripping part receives a reaction force from the body tissue and is stopped. Therefore, when body tissue that is locally protruding is incised, it is possible to incise the protruding body tissue in a direction parallel to the peripheral body tissue, thus improving the controllability of incision. Further, hemostasis is performed after incision by holding the body tissue by the gripping parts and applying high-frequency current, making it possible to continuously perform treatment without taking the treatment instrument out from the forceps hole of an endoscope.
(2) The endoscopic high-frequency treatment instrument according to (1), a second electrode is formed on a surface of the second gripping part which faces the first gripping part.

According to the endoscopic high-frequency treatment instrument, it may be possible to increase the range in which an incision can be performed and to increase the range where the electrodes are provided, by the cooperation of the first and second electrodes.
(3) The endoscopic high-frequency treatment instrument according to (1) or (2), further includes an opening/closing mechanism. The opening/closing mechanism opens the second gripping part about a position as a rotation center. The position retreats from a front end of the first gripping part toward a base end by a predetermined distance. The opening/closing mechanism opens the second gripping part with respect to the first gripping part about the rotation center at an opening angle of an obtuse angle.

According to the endoscopic high-frequency treatment instrument, the second gripping part is opened on one side with respect to the first gripping part at a right angle or an obtuse angle. Accordingly, it may be possible to increase the length of a portion of the second gripping part, which protrudes in the incision direction, so that the controllability of incision can be improved.
(4) The endoscopic high-frequency treatment instrument according to (3), a distance between the rotation center and a front end of the second gripping part is equal to or smaller than a distance between the rotation center and the front end of the first gripping part.

According to the endoscopic high-frequency treatment instrument, the length of the second gripping part is equal to or smaller than that of the first gripping part. Accordingly, the operability thereof during treatment, such as incisions, is improved.
(5) The endoscopic high-frequency treatment instrument according to any one of (1) to (4), the first electrode is formed in a linear shape along the second gripping part facing the first gripping part.

According to the endoscopic high-frequency treatment instrument, the electrode is formed in a line in order to increase the area that can be incised at one time.
(6) The endoscopic high-frequency treatment instrument according to any one of (1) to (5), the first electrode includes a protruding electrode portion which protrudes in a tapered shape from the first gripping part toward the second gripping part.

According to the endoscopic high-frequency treatment instrument, the protruding electrode protrudes from the surface of the first gripping part. Accordingly, the protruding electrode increases grip pressure when cooperating with the second gripping part so as to hold the body tissue. Therefore, the body tissue is reliably gripped and the ablation or incision using high-frequency waves is accurately performed.
(7) The endoscopic high-frequency treatment instrument according to any one of (1) to (6), the surface of the second gripping part facing the first gripping part is a non-flat surface which has concavity and convexity.

According to the endoscopic high-frequency treatment instrument, the non-flat surface having concavity and convexity such as a ridge shape can reliably grab the body tissue without slipping.
(8) The endoscopic high-frequency treatment instrument according to any one of (1) to (7), the first gripping part is made of a conductive material. An electrical insulation coating layer is formed on the outer surface of the conductive material except for the first electrode.

According to the endoscopic high-frequency treatment instrument, wires are connected not to the exposed electrodes but to a conductive material. Accordingly, current is easily supplied.
(9) The endoscopic high-frequency treatment instrument according to any one of (1) to (8), the second gripping part is made of an electrical insulation material.

According to the endoscopic high-frequency treatment instrument, movable gripping parts that do not include an electrode can be manufactured easily.

## Claims

1. An endoscopic high-frequency treatment instrument comprising:
a sheath that includes a front end, a rear end, and a longitudinal shaft;
an operation wire that is provided in the sheath so as to advance and retreat;
a first gripping part that is fixed to extend from the front end of the sheath along the longitudinal shaft;
a second gripping part that is connected to the operation wire and that is moved about a base end thereof as a rotation center so as to approach and be separated from the first gripping part as the operation wire advances and retreats with respect to the sheath; and
a first electrode that is provided on a surface of the first gripping part which faces the second gripping part.

2. The endoscopic high-frequency treatment instrument according to claim 1, wherein a second electrode is formed on a surface of the second gripping part which faces the first gripping part.

3. The endoscopic high-frequency treatment instrument according to claim 1 or 2, further comprising:
an opening/closing mechanism that opens the second gripping part about a position as a rotation center, the position retreating from a front end of the first gripping part toward a base end by a predetermined distance,
wherein the opening/closing mechanism opens the second gripping part with respect to the first gripping part about the rotation center at an opening angle of an obtuse angle.

4. The endoscopic high-frequency treatment instrument according to claim 3,
wherein a distance between the rotation center and a front end of the second gripping part is equal to or smaller than a distance between the rotation center and the front end of the first gripping part.

5. The endoscopic high-frequency treatment instrument according to any one of claims 1 to 4,
wherein the first electrode is formed in a linear shape along the second gripping part facing the first gripping part.

6. The endoscopic high-frequency treatment instrument according to any one of claims 1 to 5,
wherein the first electrode includes a protruding electrode portion which protrudes in a tapered shape from the first gripping part toward the second gripping part.

7. The endoscopic high-frequency treatment instrument according to any one of claims 1 to 6,
wherein the surface of the second gripping part facing the first gripping part is a non-flat surface which has concavity and convexity.

8. The endoscopic high-frequency treatment instrument according to any one of claims 1 to 7,
wherein the first gripping part is made of a conductive material, and
wherein an electrical insulation coating layer is formed on the outer surface of the conductive material except for the first electrode.

9. The endoscopic high-frequency treatment instrument according to any one of claims 1 to 8,
wherein the second gripping part is made of an electrical insulation material.
